(19) Europäisches Patentamt
European Patent Office
Office européen des brevets

(11) Publication number: **0 114 888 B1**

(12) **EUROPEAN PATENT SPECIFICATION**

(45) Date of publication of patent specification: **30.12.86**

(51) Int. Cl.$^4$: **C 12 Q 1/00**, C 12 Q 1/48, C 12 Q 1/52, C 12 Q 1/30, G 01 N 21/78

(21) Application number: **83902727.3**

(22) Date of filing: **03.08.83**

(86) International application number: **PCT/US83/01177**

(87) International publication number: **WO 84/00779 01.03.84 Gazette 84/06**

# (54) METHOD FOR PERFORMING RATE ASSAYS.

(30) Priority: **09.08.82 US 406629**
**27.07.83 US 517596**

(43) Date of publication of application: **08.08.84 Bulletin 84/32**

(45) Publication of the grant of the patent: **30.12.86 Bulletin 86/52**

(84) Designated Contracting States: **DE FR GB**

(56) References cited:
GB-A-2 057 684
JP-A-39 000 799
US-A-3 881 992
US-A-3 992 158
US-A-4 024 021
US-A-4 224 032
US-A-4 242 446
US-A-4 258 001
US-A-4 271 265

ANONYMOUS, RESEARCH DISCLOSURE, NUMBER 137, PAGES30-31, DISCLOSURE NUMBER 13740, SEPTEMBER 1975.

BUHL ET AL, CLINICAL CHEMISTRY,VOLUME 24, NUMBER 2, 1978, PAGES 261-268.
KLEIN, METHODS OF ENZYMATIC ANALYSIS-VOLUME 2, BERGMEYER (ED.), 1974,

(73) Proprietor: **EASTMAN KODAK COMPANY**
**343 State Street**
**Rochester New York 14650 (US)**

(72) Inventor: **WEAVER, Marilyn, Stewart**
**83 Montclair Drive**
**Rochester, NY 14617 (US)**

(74) Representative: **Baron, Paul Alexander Clifford et al**
**Kodak Limited Patent Department Headstone Drive**
**Harrow Middlesex HA1 4TY (GB)**

(56) References cited:
ACADEMIC PRESS, INC., NEW YORK, PAGES 582-585, AND PAGES 768-773.

KING, CLINICAL BIOCHEMISTRY-PRINCIPLES AND METHODS VOLUME II, CURTIUS ET AL (ED.), 1978, WALTER de GRUYTER, NEW YORK, PAGES 1148-1151.

EP 0 114 888 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a method for performing a rate assay in which an analyte activity or concentration of a test liquid is determined by the measurement of a signal that changes over time at a rate that corresponds to the analyte activity or concentration. It is particularly applicable to enzyme reactions wherein an enzyme transforms one or more substrates to produce a detectable change at a rate which is a measure of the enzyme activity.

Rate assays, and particularly enzyme assays, are based on the rate of formation or destruction of an observable signal such as a detectable density or fluorescence. The signals can be plotted as a function of time to form response curves, and the greater the amount of enzyme activity that is present, the greater the rate of change in the signal. Enzyme reaction rates tend to be most meaningful if they are uniquely determinable, for example, if they provide a linear response over time. If the signals produce a non-linear response curve, any measure of the enzyme reaction rate as the slope of the response curve will produce a non-constant value which depends upon the particular point of the curve that is considered.

Unfortunately, many rate reactions, and especially enzyme rate reactions, produce non-linear response curves, as shown, for example, in U.S.—A—4,059,405. Such non-linearity is not easily eliminated, particularly when rate reactions are measured in a test composition such as a multi-zoned element containing reagents. Non-linearity is believed to be introduced, in part, by a number of non-enzymatic, and thus extraneous, physical processes. For example, there often occurs an "induction period" in such elements in which processes extraneous to the enzyme reaction take place. These extraneous processes can extend, in certain cases, for the full length of the assay. If one could be confident that the extraneous processes were all completed by a predetermined time, then the enzyme activity could be read with confidence within a given period thereafter. However, the operator can never be certain at what time the extraneous processes are all complete. Because of this uncertainty, well-defined continuous calibration models cannot be obtained. Without proper calibration, the observable signals resulting from the enzyme reactions within the test liquid cannot be accurately and precisely converted to enzyme activity.

U.S.—A—3,847,486 discloses apparatus for performing rate assays in which a series of readings are taken of a sample, and each sample reading is followed by a blank reading. The rate of change of the difference between the sample and blank readings is used to determine the amount of a particular analyte in the sample. Although such an arrangement is useful in compensating for optical errors, the use of a blank reading precludes the use of serum as a reference liquid, since there is no known way to eliminate all enzyme activity from human serum.

The present invention overcomes the problems noted above and provides a means of linearizing a curve indicative of the increasing or decreasing detectable signals produced by a rate reaction over at least a portion of the reaction time.

In accordance with the present invention, there is provided a method for performing a rate assay in which an analyte activity or concentration of a test liquid is determined by the measurement of a signal that changes over time at a rate that corresponds to the analyte activity or concentration, the method including the steps of taking a series of measurements at constant intervals over a predetermined time of said signal, to form a first set of values, said method characterized by the steps of taking a second series of measurements at said intervals over a time equal to said predetermined time of a signal which results from a reference liquid of a predetermined above-zero activity or concentration and which changes over time at a rate that corresponds to the analyte activity or concentration of the reference liquid, to form a second set of values in which each of the values corresponds to a value in said first set, subtracting each value in said second set of values from the corresponding value in said first set of values to produce an adjusted set of values, and determining the analyte activity or concentration in said test liquid based on the rate of change of said adjusted set of values.

One advantage of the present invention is that a reliable reaction rate is readily ascertainable, even when the response curves of the "raw" detectable signals produced by the test liquids being assayed are grossly non-linear. Another advantage of the invention is that such reliable reaction rates are determinable when using multi-layered test elements in which non-enzymatic physical processes can occur. A further advantage of the invention is that greater linearity permits the use of a longer time period for evaluation in the enzyme assay; this in turn reduces the likelihood of a large protein bias, which occurs usually at the early stages of the reaction for enzymes such as LDH, ALT and AST, and it further reduces the likelihood that instrument noise will be a significant factor in the test results.

Embodiments of the present invention will now be described, by way of example, with reference to the accompanying drawing in which:

Fig. 1 is a schematic illustration of an analyzer which can be used to perform the present invention;

Fig. 2 is a schematic illustration of the computing means and photodetector of the analyzer;

Figs. 3A and 3B are logic flow charts for the programming of the analyzer computing means;

Fig. 4 shows the detectable signals produced by one enzyme in the form of reflection density plotted against time;

Fig. 5 shows the graphs of Fig. 4 after

subtraction of the reflection density values generated by a reference liquid;

Figs. 6—7 and 8—9 are two sets of graphs similar to those of Figs. 4 and 5, for certain enzyme assays;

Figs. 10 and 11 also are graphs similar to those of Figs. 4 and 5, except that a reference liquid having a high level of enzyme activity was used for the subtraction step;

Figs. 12 and 13 are a pair of graphs similar to those of Figs. 4 and 5, except that the calibrators were prepared using non-human serum; and

Figs. 14 and 15 are a pair of graphs similar to those of Figs. 4 and 5, except that the enzyme illustrated is ALT.

The present invention is described hereinafter in connection with a test composition that is a multi-zoned test element. In addition, the invention is useful with other test compositions, particularly if such compositions tend to produce non-linear rate reactions. The present invention is also described in connection with the determination of enzyme activity levels. In addition, the invention is applicable to the determination of any analyte concentration, particularly those that produce a raw signal that varies non-linearly with respect to time.

A variety of test elements constructed to conduct a rate assay, such as an enzyme assay, are useful with the present invention. Preferred are multi-zoned elements having a plurality of reagents and functions that are divided among the zones. Most preferred are elements in which the zones are separate but contiguous layers, for example, a multi-layered test element as described in U.S.—A—3,992,158 and in U.S.—A—4,258,001. The test elements described in these patents include an uppermost layer that functions to transport the liquid to be tested to the next adjacent layer or layers. Such uppermost layer optionally includes a reagent for the test, for example, one or more substrates for the enzyme. The next adjacent layer or layers preferably include a matrix or binder and additional reagents for the assay. These additional reagents include those necessary to produce a detectable signal in increasing amounts, for example, a reflection density or fluorescence, in response to the reaction of the enzyme. Alternatively, such reagents produce a detectable signal which is destroyed or converted to a non-detectable species at a rate proportional to the amount of enzyme present. The detectable signal is inclusive of, but not limited to, reflectances and luminescent signals such as a fluorescent signal. Other detectable signals are useful as well, for example, potentiometric signals. In the discussion which follows, density-producing or density-destroying enzymatic reactions are emphasized as the most preferred reactions.

The present invention is particularly useful in assaying a dehydrogenase or a dehydrogenase-coupled enzyme reaction, using test elements containing NADH as a coenzyme.

A highly preferred test element comprises, as the uppermost layer, a non-fibrous, isotropically porous spreading layer such as a layer comprising microcrystalline cellulose, for example, that which is available from FMC Corporation under the trademark "Avicel", a binder such as polyvinylpyrrolidone, a buffer that maintains an optimum pH for the enzyme, for example, about 7.2, and a substrate for the enzyme being assayed. The adjacent layer in such an element preferably comprises a hardened gelatin binder, a surfactant, a buffer that maintains an optimum pH and a color-producing or colored species such as NADH. Such layer is disposed on a transparent support, such as a plastic or glass.

Using test elements constructed as described above, this invention is particularly useful in assaying for lactate dehydrogenase (LDH). In that usage, the substrate that is incorporated into the uppermost layer is sodium pyruvate. When LDH encounters this substrate, the enzyme catalyzes the following reaction:

$$\text{sodium pyruvate} + \text{NADH} + \text{H}^{\oplus} \xrightarrow{\text{LDH}} \text{L-lactate} + \text{NAD}^{\oplus}$$

Alternatively, the above-noted reagents, sodium pyruvate and NADH, are useful if disposed all in one layer, for example, in the uppermost layer, or in a single-zone test element. Preferably, in such an element, the matrix is the same as is described for the uppermost layer in the previous paragraph. For best results in assaying LDH, such an element is used fresh, rather than after extensive storage.

The present invention is useful in any analyzer constructed to repetitively examine each test composition rapidly so that the rate of increase or decrease of the detectable signal of each test composition can be determined. One such analyzer is described in U.S.—A—4,224,032. In Fig. 1, there is shown an analyzer 10 for use in performing the present invention. Analyzer 10 comprises a test liquid supply means 20, a test element supply means 30, metering device 40, incubator 50, an element transfer mechanism 45 for transferring a test element from supply means 30 to metering device 40 and thence to incubator 50, a photometer 70, and a computing means 100. Analyzer 10 also includes an incubator 130 and a means for radiometric analysis 132 for use when different kinds of tests are being performed.

Supply means 20 comprises a rotatable tray 22 containing a plurality of disposable containers 24 and means for rotating the tray 22 (not shown). Test element supply means 30 comprises a rotatable table 32 which supports a plurality of stacks A, B, C, and D of test elements, and drive means for rotating the table 32 so that one or another of the stacks of test elements is presented to an ejector mechanism 34 which feeds them to transfer mechanism 45. Metering device 40 is adapted to lift one of the containers 24 to a pressurizing means, the containers 24 having a dispensing aperture (not shown) which permits the formation of a pendant drop of liquid in

response to the pressure generated by the pressurizing means. Transfer mechanism 45 comprises a rotatable arm 90 which positions a test element under such pressurized container, at metering device 40, to contact the drop to the test element.

Incubator 50 (Fig. 1) comprises a rotor 52 provided with multiple stations each adapted to receive a test element, a drive shaft 54, and rotor drive means 56. Drive means 56 is adapted to rotate rotor 52 at a fixed rate, for example, 12 RPM.

Photometer 70 comprises a light source 72, optics for directing light as a beam 76 into the interior of rotor 52 while it rotates, and a photodetector 78. As is conventional, beam 76 is inclined at 45° to the normal of the test elements, while radiation is detected therefrom as diffuse radiation reflected along the normal, as indicated by arrows 80. Radiation is reflected to photodetector 78 by, for example, mirror 82 disposed inside rotor 52. Filters (not shown) are placed, if desired, either between light source 72 and the test element in rotor 52, or between such test element and photodetector 78.

The signal generated by photodetector 78 is directed to computing means 100 which functions with control center 102 to control the operations of the analyzer 10. As is conventional, such computing means 100 further includes input/output devices, such as keyboard 104 and display 106.

Computing means 100 is programmed to collect, via an amplifier 91 and analog-to-digital converter 92 (Fig. 2) the values representing, for the preferred embodiment, the reflection densities of the test elements containing a test liquid, and the reflection densities of a test element containing a reference liquid having a predetermined enzyme activity. As used herein, the term "reference liquid" means a liquid having any predetermined enzyme activity level above zero activity; even high activity levels are useful in the reference liquid. Any suitably programmed digital computer is useful in the invention. Preferred are analyzers containing a programmable microprocessor 100' (Fig. 2) which comprises a central processing unit 200, for example, an Intel 8086 chip, and memory units 202 comprising one or more RAM's 204 and optionally one or more E PROM's 208.

In accordance with the process of the present invention, the set of values determined as representing the detectable signals generated by the test liquids in the test elements are processed by subtracting therefrom the corresponding value in the set of values representing the detectable signals of an identical test element containing a reference liquid. It is the adjusted value obtained from this subtraction, that is used to establish the rate of change of the reaction. The values representing the detectable signals are preferably reflection densities or reflectances, where reflection density $D_R=-\log_{10}$ (reflectance). In carrying out this process, the "raw" density calculated from the reflectance actually detected at each scanning time is used, or, a plurality of replicates, closely-spaced in time, are taken from which a mean value and the corresponding density are calculated. To use such a technique, photometer 70 provides multiple readings each time a test element is scanned. Each of these readings represents the density of a fraction of the total density-producing area of the element exposed to beam 76. More specifically, the procedure is to scan at time $t_1$ the density-producing portion of the test element, and integrate the light detected for that area. This integrated signal is amplified at 91 and sent to the A/D converter 92 (Fig. 2) where a plurality of digital values are obtained from the integrated signal. These digital values are averaged to obtain the mean density value for time $t_1$. At time $t_2$, which is, for example, 5 seconds later, the process is repeated. The programming of computer means 100 or 100' to determine such values is conventional.

If mean values are used to obtain reflectances or reflection density values generated by the test liquid, preferably mean values are similarly ascertained in order to determine reflectances or reflection density values generated by the reference liquid.

To convert reflectance signals to reflection densities, the following equation is used by the computing means:

$$1) \qquad D_R=-\log_{10}[(S_{A/D}-\delta_{A/D})/(R_{A/D}-\delta_{A/D})]$$

where $S_{A/D}$ is the A to D conversion value taken for the light intensity of either the reference liquid or the test liquid, $R_{A/D}$ is such A to D conversion value for a white reference that is part of the analyzer, and $\delta_{A/D}$ is the A to D conversion value for the light intensity produced by a dark or black reference that is part of the analyzer.

In some enzyme chemistries, for example, γ-glutamyltransferase and alkaline phosphatase, transmission densities ($D_T$) are preferred. In that case, a separate branching routine is followed by the computing means to convert $D_R$ to $D_T$. Such conversion is described in 24 *Clin. Chem.,* pp. 1335—1342, and especially p. 1340, (1978), except that the conversion equation set forth in the article should be

$$2) \; D_T=-0.194+0.469\,D_R+0.422/(1+1.179e^{3.379D_R}).$$

Optionally, and particularly if the detectable signals determined for the reference liquid are greater than those of the test liquid, a constant value is added back to each of the difference values to convert all readings into positive values.

It has been found that, once the subtraction of the reference liquid $D_R$ (or $D_T$) values has occurred, for many chemistries the resulting values are linear over extended periods of time so that the constant rate of change is readily ascertained. Any conventional technique is useful for ascertaining or approximating the slope of such linear data. In one useful technique, starting with

any appropriate point on the curve, for example, 60 sec., the computing means compares the rate for each time $t_i$ of scanning ($t_1$, $t_2$, etc., from the above example) against the rate of the previous time $t_{i-1}$, and determines if the rate of change is changing beyond a predetermined limit (for example, ±5%). When the predetermined limit is reached, the rates obtained thereafter are discarded, and the rates obtained prior to reaching the limit are then averaged to produce an overall approximation. Conventional subroutines and apparatus, such as microprocessor 100', are used to carry out the approximating step described above.

In the aforedescribed process, the values representing the reflectances or reflection densities generated by the reference liquid are determined either before, during, or after the readings of the test liquids. In any event, the reference liquid values are preferably stored in the memory units of computing means 100 or microprocessor 100'.

Most preferably, the values for the reference liquid are determined during calibration. That is, it is a practice to calibrate on a periodic basis, for example, once a week. In this procedure, calibrator liquids having known enzyme activity levels are tested for reflection density or reflectance responses. The procedure of the invention described above is applied to the calibrator liquids, so that each rate of change that is finally ascertained, after subtracting the reference liquid's reflection density responses, is assignable to a known enzyme activity. It is during this procedure that the reference liquid is preferably tested. For example, the calibrator containing the lowest level enzyme activity is useful as the reference liquid. As will be readily appreciated, such a reference liquid is particularly preferred if a change in the sign of the rate is to be avoided when the calibration curve is prepared.

In preparing a calibration curve as a plot of enzyme international units per liter (U/L) versus rate, the reference liquid, following subtraction, produces a zero rate or slope for the enzyme activity chosen as the reference level. The "zero-rate" data point thus obtained for the reference liquid is used together with the known enzyme activity of the reference liquid, as one of the data points from which the calibration curve is regressed.

Figs. 3a and 3b illustrate logic flow charts that are useful in programming microprocessor 100' to perform the aforedescribed process. From these flow charts a program routine is readily determinable using conventional programming techniques. Specifically, subscripted variable $t_i$ represents the read times at which the reflectances and eventually reflection density values are ascertained. As shown in box 300, (Fig. 3a) the readings are taken over n time units, using m intervals, for example, 5-second intervals. Any start time is useful, it being assumed here that i=1 represents the first second after a preliminary, non-reading period. At each $t_i$, the reflectance is read for the reference liquid (box 302). The read values are stored in computing means 100 or 100' as values $RL_i$ (box 304). Other calibration steps then proceed, whereby a calibration curve of rate of change of reflection density versus enzyme activity is established as internal data within computing means 100 (or 100').

As an additional part of the process of the invention, each test liquid, including any non-reference calibrator liquids used for calibration, is examined for its detectable signal values (for example, reflectance) at each $t_i$, the same times being used as were used for the reference liquid. Thus, as shown in boxes 310 and 312 of Fig. 3b, the same preliminary, non-reading period is used and the readings taken at the same start time and at the same m intervals, for example, 5 seconds, as were used in examining the reference liquid. The stored value $RL_i$ of the reference liquid is retrieved for each of these times, as indicated in box 313. Before the subtraction step of the invention is applied, in one embodiment of the invention the values to be modified, for example, reflectance values, are all converted (box 314) to reflection density values, $D_R$, as described above. If the enzyme is preferably evaluated as a transmission density (box 315) then the logic branches to a step that converts each $D_R$ to $D_T$, as described above. Thereafter, each $D_R$ (or $D_T$) so obtained from $RL_i$ is subtracted from each corresponding read value of the test liquid (box 316). The subtraction step is preferably done by the programmed computing means 100 or microprocessor 100'. When this subtraction is completed for all times $t_i$, optionally the program branches, as shown by the dashed lines, to examine the difference values to ascertain if any value is less than zero (box 318). In such a case, since there is no real negative density, it is useful to add a constant (the same constant for all subtractions) in an amount such that all difference values are non-negative (box 320).

After all times $t_i$ have been processed, the density values modified by the subtraction step as just described are optionally stored (box 330) and subsequently operated on by the subroutine for evaluating the slope.

The foregoing process is repeated for each test liquid.

Equivalently, reflectance rather than reflection densities are useful as the values to be applied during the linearizing step. In such case, since subtraction of two logs equals the log of the quotient of the arguments within the two logs, the reflectance values of each test liquid are each divided by the respective reflectance values of the reference liquid for the corresponding time $t_i$ of the reaction, and the negative $\log_{10}$ of the quotient is the value to be examined, at each time $t_i$, for slope determination.

The composition of the reference liquid providing the values used in the subtraction step, is not critical. For example, any source of the enzyme is useful. Any matrix is useful, provided it is compatible with the test liquid.

The examples which follow further illustrate the present invention.

Example 1

Multilayered analytical elements for the determination of lactate dehydrogenase were prepared as follows:

A sheet of subbed poly(ethylene terephthalate) was coated with a reagent layer comprising deionized gelatin, 0.43 $g/m^2$ (4.8 mMolar) of NADH, N - [2 - hydroxy - 1,1 - bis(hydroxymethyl)ethyl]taurine buffer (hereinafter TES), octylphenoxy polyethoxy ethanol (a non-ionic surfactant) and a gel hardening agent. Over this was coated a spreading layer comprising microcrystalline cellulose obtained from FMC Corp. under the trademark "Avicel", poly(vinyl pyrrolidone) obtained from GAF Corp., the TES buffer noted above, and 0.1 $g/m^2$ (7.5 mMolar) of sodium pyruvate.

Onto each element was metered a 10 µl drop of calibration liquid comprising human serum and a predetermined level of LDH obtained from rabbit muscle, namely, one of the activity levels 8, 217, 437, 547, 605, 1134, 1685, 2376 and 3071 U/L. These enzyme activities were determined using as a reference assay the procedure described in *Clin. Chem.*, Vol. 24, No. 2, pp. 261—266 (1978) modified for the Rotochem Analyzer.

The test elements were placed in an incubator that maintained the temperature at 37°C. Each of these elements containing the calibrator was examined every 5 seconds ($t_i$) in an analyzer similar to that shown in Fig. 1. (Occasionally a data point may not be read at a particular enzyme level, and in such a case the value for that time $t_i$ is interpolated). A plurality of reflectance readings were taken at each $t_i$ and a mean value of reflectance ascertained. Four replicates of each liquid were used following this procedure, and averaged. For comparative purposes, the $D_R$ values for these reflectances were obtained as the negative $\log_{10}$ of the reflectances, and plotted to give markedly non-linear curves of Fig. 4. Although the curves are shown as being continuous, they were in fact the 5 sec. values connected point to point by straight lines. The numbers at the right-hand ends of the curves identify the enzyme activity level.

Next, as the reference values, each of the reflectance values so obtained for the calibrator containing 8 U/L of LDH was divided into the reflectance values obtained for the other calibrators at the corresponding times $t_i$, to give modified values every five seconds. The negative $\log_{10}$ was taken of these modified values and when sequentially connected by straight lines, the resulting $D_R$ values formed the curves of Fig. 5. The enzyme levels again are listed at the right-hand ends of the curves. Although the reference having an activity of 8 U/L was not actually plotted, if it were it would appear as the dashed horizontal straight line shown.

It will be readily apparent that, following the subtraction step, the reflection density profiles for all enzyme levels were more linear than the reflection density profiles of Fig. 4. Of particular interest is the fact that the four levels 217, 437, 547 and 605 U/L were substantially linear for their entire range.

The linearized curves of Fig. 5 are readily measured for slope, or rate of change, by the techniques noted above. In contrast, any attempt to use the curves of Fig. 4 produces a non-constant and therefrom non-unique result.

Because of the superior linearity of the curves of Fig. 5, the dynamic range that is readily detectable is expanded to include higher levels of enzyme activity. Such higher levels heretofore were difficult to assay because of the non-linearity of their curves. Furthermore, in the results of Fig. 5, the analyzer can make its reading with precision anywhere between 15 and 75 sec., regardless of the particular enzyme activity level. However, using the curves of Fig. 4, the curvature at 60 sec. for the low levels of activity would prevent the rate from being uniquely determined at the 60 sec. time, for example.

Most preferably, however, this invention allows the analyzer to read as far out as linearity remains in the data, following subtraction of the reference liquid's values. For example, the slope can be examined over the time 15 sec. to about 4 minutes, for LDH activity of 605 U/L, without detecting a sufficient departure from linearity as to stop the slope calculation. However, in the case of LDH at 3071 U/L, the slope can be measured only out to about 90 sec. before curvature causes the slope to change at a rate exceeding preassigned limits. This ability to read the slope for as long as linearity is present permits the rate to be read over a longer time, particularly at lower enzyme activity levels. This is advantageous because the imprecision due, for example, to instrument noise, is thereby reduced.

Example 2

Use in assaying for LD-4 isoenzyme

The procedure of Example 1 was repeated, except that the calibrator liquids metered onto the test elements each contained various predetermined levels of LD-4 isoenzyme obtained from human sources, having the activities 11, 110, 386, 788, 1458 and 2223 U/L. The response values representing the corresponding reflection densities appear in Fig. 6 as markedly non-linear curves. To improve these curves, each of the 5-second interval reflectance values of the curve for the 11 U/L activity were divided, as reference values, into the reflectance values for the corresponding 5-second reading of the other curves. The negative $\log_{10}$ of these modified values produced modified $D_R$ values, which appear in Fig. 7. It will be readily apparent that, following the subtraction step, the reflection density profiles for all enzyme levels were more linear, which is in marked contrast to the profiles of Fig. 6. The difference values for the 110 and 386 U/L activity are substantially linear for the entire range.

Example 3

Use in assaying for AST

The procedure of Example 1 was repeated, except that the test elements were constructed to assay for aspartate aminotransferase (AST) as follows:

A polyethylene terephthalate film was coated with a reagent layer comprising deionized gelatin, surfactant, gel hardening agent, tris(hydroxymethyl)aminomethane (tris) buffer, NADH (0.32 $g/m^2$ or 3.6 mMolar), pyridoxal - 5 - phosphoric acid (PLP) (0.16 $g/m^2$), malate dehydrogenase (MDH) and lactate dehydrogenase (LDH); a subbing layer comprising poly(N - isopropylacrylamide); and a spreading layer comprising barium sulfate, cellulose acetate, the non-ionic surfactant of Example 1, a polyurethane resin elastomer obtained from B. F. Goodrich, sodium α-ketoglutarate and sodium aspartate.

The calibrator liquids comprised a bovine serum albumin matrix in which the AST levels were adjusted using porcine heart AST, so that samples had one of the following predetermined levels of AST activity: 25, 641, 1246, 2316, and 4777 U/L. Fig. 8 is a plot of the $D_R$ response curves for the reflectances originally detected. Thereafter, each of the 5-second reflectance values of the curve for the 25 U/L activity, the reference liquid, was divided into the reflectance values obtained at the corresponding time for the other curves. When converted into reflection densities the resulting modified values appear in Fig. 9. Again, marked improvement in linearity was noted.

Example 4

Use of a high level enzyme activity reference liquid

The procedure of Example 1 was repeated, except that a calibrator liquid having 1292 U/L activity of LDH was selected as the reference liquid. Before modifying the reflectance values using the reflectances obtained for the reference liquid, the reflection density response curves were as shown in Fig. 10. After division of reflectances (equal to subtraction of reflection densities), the corresponding reflection density curves appeared as shown in Fig. 11. Even those levels above 1292 U/L demonstrated, after subtraction, a longer time span of linearity than was available before subtraction. For example, the highest level at 3840 U/L was generally linear after subtraction out to 1.5 minutes, whereas before it was generally linear only out to 1.0 minute.

In carrying out the aforedescribed analysis of the slope of the curves of Fig. 11, the tail portions labelled "B'", for levels 2425, 3320 and 3840 U/L, would of course not be used, as they represent the reactant depletion region.

Example 5

Use of a non-human reference liquid

Essentially the procedure of Example 1 was repeated except that the test element formulation was that of Example 2, and the calibrators were prepared from a bovine serum pool that was stripped of LDH and spiked with LDH obtained from porcine heart to achieve the desired levels. Fig. 12 is the graph of the response curves of the calibrators before the reference liquid's values were subtracted, point-by-point. Fig. 13 is the corresponding graph produced following subtraction of the values for the 16 U/L activity, selected to be the reference liquid. As before, the linearity was markedly improved by the subtraction step.

Example 6

The procedure of Example 1 was repeated, except that the LDH samples tested were actual patient serum samples, rather than serum-based calibrators. A marked improvement in linearity was observed in the data after subtraction of the reference liquid's values, point-by-point, just as in the case of Example 1.

Example 7

The procedure of Example 4 was repeated, except that the reflection density $D_R$ values for the enzyme activity 341 U/L, Fig. 10, were divided by the reflection density $D_R$ values for the liquid having an activity level of 45 U/L, as the reference liquid for this example. Thus, the arithmetic operation was division, whereas in Examples 1—6 previously described, the operation was subtraction. The resulting plot of the modified $D_R$ was more linear than the original plot for 341, Fig. 10.

Example 8

Use in assaying for alanine aminotransferase (ALT)

A procedure similar to that of Example 3 was followed, except that malate dehydrogenase was omitted from the reagent layer, the subbing layer was poly(vinyl pyrrolidone), and the spreading layer included L-alanine in place of sodium aspartate.

Onto each element was metered a 10 µl drop of calibration liquid comprising human serum and a predetermined level of ALT obtained from porcine heart, namely, one of the activity levels 0.5, 22.7, 42.7, 70.4, 170.2, 306.6, 506.1, 674.0, and 899.1 U/L. These enzyme activities were determined using as a reference assay the IFCC recommended procedure modified for the COBAS Analyzer. The test elements were placed in an incubator that maintained the temperature at 37°C. Each of these elements containing the calibrator was examined every 6 seconds ($t_i$) in an analyzer similar to that shown in Fig. 1. (Occasionally a data point may not have been read at a particular enzyme level, and in such a case the value for that time $t_i$ was interpolated). A plurality of reflectance readings were taken at each $t_i$ and a mean value of reflectance ascertained. Nine replicates of each fluid were used following this procedure, and averaged. For comparative purposes, the $D_R$ values for these reflectances were obtained as the negative $\log_{10}$

of the reflectances, and plotted to give markedly non-linear curves of Fig. 14. Although the curves areshown as being continuous, they were in fact the 6 sec. values connected point to point by straight lines. The numbers at the right-hand ends of the curves identify the enzyme activity level.

Next, as the reference values, each of the reflectance values so obtained for the calibrator containing 0.5 U/L of ALT was divided into the reflectance values obtained for the other calibrators at the corresponding times $t_i$, to give modified values every six seconds. The negative $\log_{10}$ was taken of these modified values and when sequentially connected by straight lines, the resulting $D_R$ values formed the curves of Fig. 15. The enzyme levels again are listed at the right-hand ends of the curves.

It will be readily apparent that, following the subtraction step, the reflection density profiles for all enzyme levels of Fig. 15 were more linear than the reflection density profiles of Fig. 14.

This example further demonstrates the advantage of using a non-zero enzyme activity reference liquid for the subtraction step. That is, the 0.5 U/L reference liquid was obtained by diligently attempting to completely eliminate the ALT activity in the human serum by pH treatment. Notwithstanding the attempt, it was not possible to eliminate all activity—a known phenomenon using serum-based liquids. If a zero activity were desired, a non-serum matrix such as albumin would have had to be used. However, the other examples stated heretofore clearly indicate that such zero activity is unnecessary inasmuch as any non-zero enzyme activity level is useful. The ability to use non-zero enzyme activities means that a much broader field of reference liquids can be used than otherwise would be the case, namely the serum-based liquids.

## Claims

1. A method for performing a rate assay in which an analyte activity or concentration of a test liquid is determined by the measurement of a signal that changes over time at a rate that corresponds to the analyte activity or concentration, the method including the steps of taking a series of measurements at constant intervals over a predetermined time of said signal, to form a first set of values, said method characterized by the steps of taking a second series of measurements at said intervals over a time equal to said predetermined time of a signal which results from a reference liquid of a predetermined above-zero activity or concentration and which changes over time at a rate that corresponds to the analyte activity or concentration of the reference liquid, to form a second set of values in which each of the values corresponds to a value in said first set, subtracting each value in said second set of values from the corresponding value in said first set of values to produce an adjusted set of values, and determining the analyte activity or concentration in said test liquid based on the rate of change of said adjusted set of values.

2. A method according to claim 1, characterized in that said assay is a measure of enzyme activity.

3. A method according to claim 2, characterized in that said enzyme activity is selected from the group consisting of dehydrogenase activity and aminotransferase activity.

4. A method according to claim 2, characterized in that said enzyme activity is lactate dehydrogenase activity.

5. A method according to Claim 2, characterized in that said enzyme activity is aspartate aminotransferase activity.

6. A method according to Claim 2, characterized in that said enzyme activity is alanine aminotransferase activity.

7. A method according to any of the preceding claims, characterized in that said arithmetic operation comprises the step of subtracting each value in one set from the corresponding value in the other set.

8. A method according to any of the preceding claims, characterized in that a predetermined number of replicates are performed for each of said measurements, and a mean of the replicates for each measurement is determined prior to determining the respective set of values.

9. A method according to any of the preceding claims, characterized in that said liquids are deposited on test elements and said elements produce said signals.

10. A method according to any of the preceding claims, characterized in that the set of values determined for said reference liquid are stored for use in said arithmetic operation.

## Patentansprüche

1. Verfahren zur Ausführung von Geschwindigkeitsanalysen, bei dem eine Analyt-Aktivität oder Analyt-Konzentration einer Test-Flüssigkeit durch Messung eines Signals bestimmt wird, das sich im Verlaufe der Zeit mit einer Geschwindigkeit verändert, die der Analyt-Aktivität oder Analyt-Konzentration entspricht, wobei das Verfahren die Stufen der Durchführung einer Reihe von Messungen in konstanten Zeitabständen über eine vorbestimmte Signaldauer umfaßt, um einen ersten Satz von Werten zu erhalten, gekennzeichnet durch die Stufen der Durchführung einer zweiten Reihe von Messungen in den Zeitabständen über einen Zeitraum, der gleich ist der vorbestimmten Dauer eines Signals, das von einer Bezugsflüssigkeit einer vorbestimmten Übernull-Aktivität oder Übernull-Konzentration erhalten wird und das sich im Verlaufe der Zeit mit einer Geschwindigkeit verändert, die der Analyt-Aktivität oder Analyt-Konzentration der Bezugsflüssigkeit entspricht, zur Bildung eines zweiten Satzes von Werten, in dem jeder Wert einem Wert des ersten Satzes entspricht, Subtraktion eines jeden Wertes des zweiten Satzes von Werten von dem entsprechenden Wert des ersten Satzes von Werten unter Bildung eines

berichtigten Satzes von Werten und Bestimmung der Analyt-Aktivität oder Analyt-Konzentration in der Test-Flüssigkeit aufgrund der Geschwindigkeit der Veränderung des berichtigten Satzes von Werten.

2. Verfahren nach Anspruch 1, dadurch gekennzeichnet, daß die Analyse aus einer Messung einer Enzym-Aktivität besteht.

3. Verfahren nach Anspruch 2, dadurch gekennzeichnet, daß es sich bei der Enzym-Aktivität um eine Dehydrogenase-Aktivität oder Aminotransferase-Aktivität handelt.

4. Verfahren nach Anspruch 2, dadurch gekennzeichnet, daß es sich bei der Enzym-Aktivität um eine Lactatdehydrogenase-Aktivität handelt.

5. Verfahren nach Anspruch 2, dadurch gekennzeichnet, daß es sich bei der Enzym-Aktivität um eine Aspartataminotransferase-Aktivität handelt.

6. Verfahren nach Anspruch 2, dadurch gekennzeichnet, daß es sich bei der Enzym-Aktivität um eine Alaninaminotransferase-Aktivität handelt.

7. Verfahren nach einem der vorstehenden Ansprüche, dadurch gekennzeichnet, daß die arithmetische Operation die Stufe der Subtraktion eines jeden Wertes eines Satzes von dem entsprechenden Wert des anderen Satzes umfaßt.

8. Verfahren nach einem der vorstehenden Ansprüche, dadurch gekennzeichnet, daß im Falle jeder Messung eine vorbestimmte Anzahl von Wiederholungen erfolgt und daß für jede Messung ein Mittelwert aus den Wiederholungen ermittelt wird, bevor der entsprechende Satz von Werten ermittelt wird.

9. Verfahren nach einem der vorstehenden Ansprüche, dadurch gekennzeichnet, daß die Flüssigkeiten auf Testelementen abgeschieden werden, und daß die Signale von diesen Elementen erzeugt werden.

10. Verfahren nach einem der vorstehenden Ansprüche, dadurch gekennzeichnet, daß der Satz von Werten, der für die Bezugsflüssigkeit ermittelt wird, zum Zwecke der Verwendung in der arithmetischen Operation gespeichert wird.

**Revendications**

1. Procédé pour effectuer une analyse basée sur la vitesse, dans laquelle l'activité ou la concentration du constituant à doser dans un liquide d'analyse est déterminée par la mesure d'un signal variant dans le temps à une vitesse qui est fonction de l'activité ou de la concentration du constituant à doser, le procédé comprenant l'étape de prise à intervalles réguliers pendant une période de temps déterminée d'une série de mesures dudit signal, pour former un premier ensemble de valeurs, ledit procédé étant caractérisé par l'étape de prise auxdits intervalles réguliers pendant une période de temps égale à ladite période de temps déterminée d'un signal provenant d'un liquide de référence d'activité ou de concentration déterminée non nulle, variant dans le temps à une vitesse qui est fonction de l'activité ou de la concentration du constituant à doser dans le liquide de référence, pour former un deuxième ensemble de valeurs dans lequel chaque valeur correspond à une valeur dudit premier ensemble, par l'étape de soustraction de chaque valeur du deuxième ensemble de la valeur correspondante du premier ensemble pour obtenir un ensemble de valeurs corrigé et déterminer l'activité ou la concentration du constituant à doser dans ledit liquide d'analyse en fonction de la vitesse à laquelle ledit ensemble de valeurs corrigé varie.

2. Un procédé selon la revendication 1, caractérisé en ce que ladite analyse est une mesure de l'activité enzymatique.

3. Un procédé selon la revendication 2, caractérisé en ce que ladite activité de l'enzyme est choisie dans le groupe comprenant l'activité du déshydrogénase et de l'amino-transférase.

4. Procédé selon la revendication 2, caractérisé en ce que ladite activité de l'enzyme est l'activité du lactate déshydrogénase.

5. Procédé selon la revendication 2, caractérisé en ce que ladite activité de l'enzyme est l'activité de l'aspartate amino-transférase.

6. Procédé selon la revendication 2, caractérisé en ce que ladite activité de l'enzyme est l'activité de l'analine amino-transférase.

7. Procédé selon l'une quelconque des revendications précédentes, caractérisé en ce que l'opération arithmétique comprend l'étape de soustraction de chaque valeur d'un ensemble de valeurs de la valeur correspondante de l'autre ensemble de valeurs.

8. Procédé selon l'une quelconque des revendications précédentes, caractérisé en ce que l'on répète un nombre déterminé de fois chacune desdites mesures, et en ce que le moyen de répéter chacune de ces mesures est déterminé avant de déterminer les ensembles de valeurs respectifs.

9. Procédé selon l'une quelconque des revendications précédentes, caractérisé en ce que l'on dépose lesdits liquides sur des produits analytiques, et que lesdits produits analytiques produisent lesdits signaux.

10. Procédé selon l'une quelconque des revendications précédentes, caractérisé en ce que l'ensemble de valeurs déterminé pour chaque liquide de référence est mis en mémoire pour être utilisé dans ladite opération arithmétique.

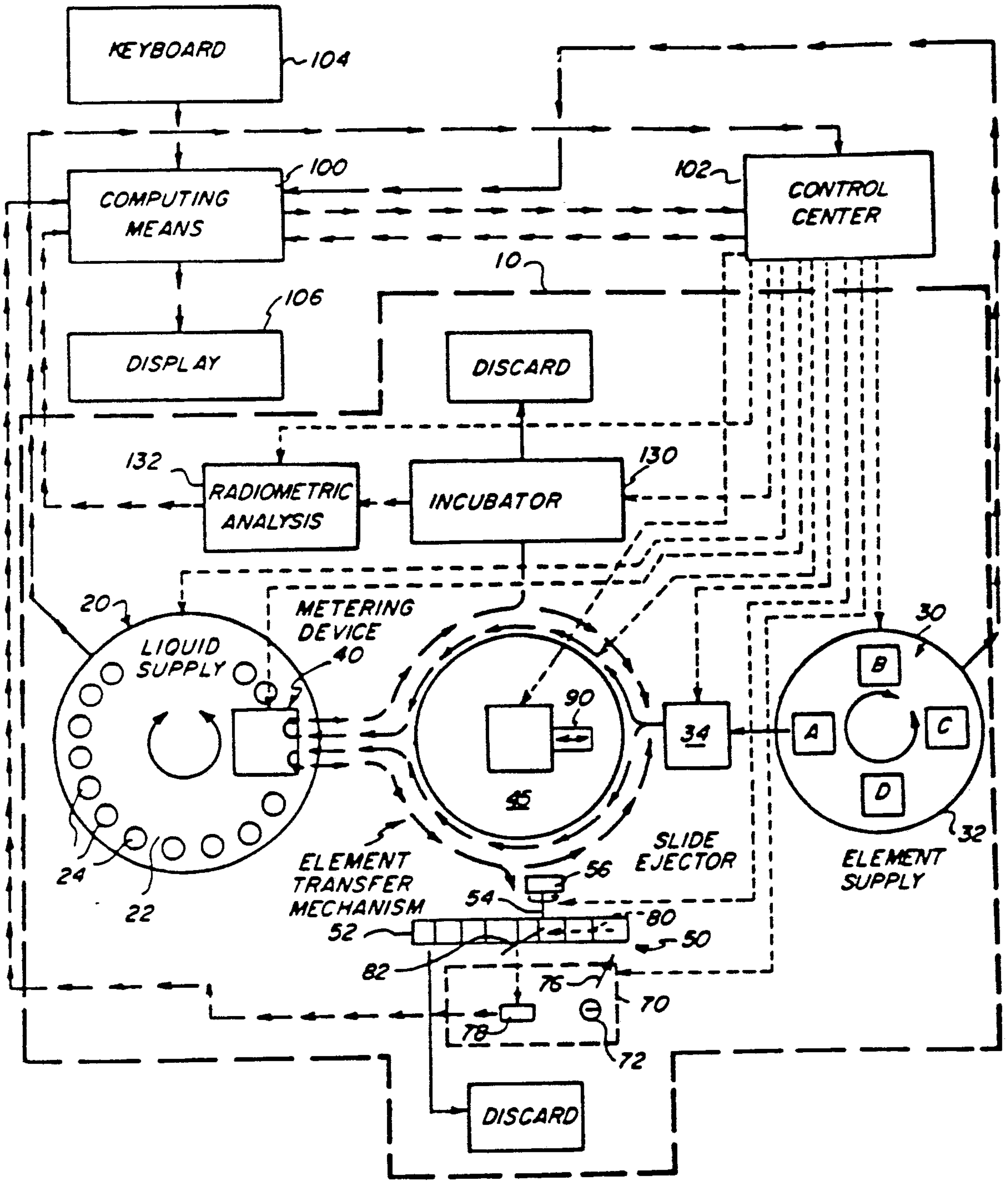
KEYBOARD
104
COMPUTING MEANS
100
102
CONTROL CENTER
10
106
DISPLAY
DISCARD
132
RADIOMETRIC ANALYSIS
INCUBATOR
130
20
LIQUID SUPPLY
METERING DEVICE
40
30
B
A
C
D
34
90
45
56
SLIDE EJECTOR
ELEMENT SUPPLY
32
24
22
ELEMENT TRANSFER MECHANISM
54
52
80
50
82
76
70
78
72
DISCARD

FIG. 1

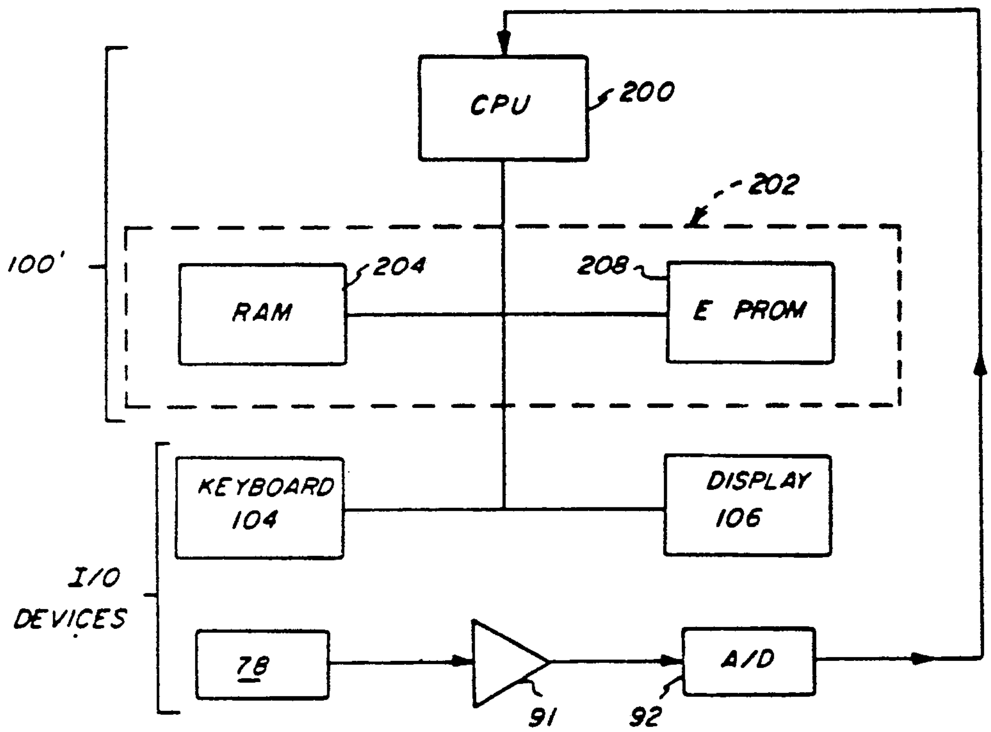
CPU
200
202
100'
RAM
204
208
E PROM
KEYBOARD
104
DISPLAY
106
I/O
DEVICES
78
A/D
91
92

FIG. 2

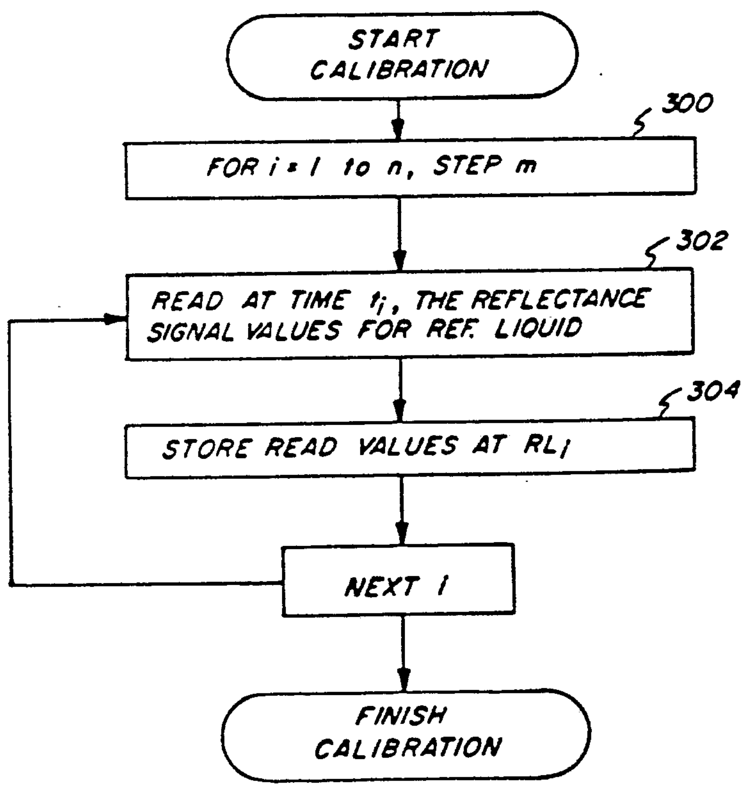
START
CALIBRATION
300
FOR i = 1 to n, STEP m
302
READ AT TIME $t_i$, THE REFLECTANCE
SIGNAL VALUES FOR REF. LIQUID
304
STORE READ VALUES AT $RL_i$
NEXT i
FINISH
CALIBRATION

FIG. 3a

FIG. 3b

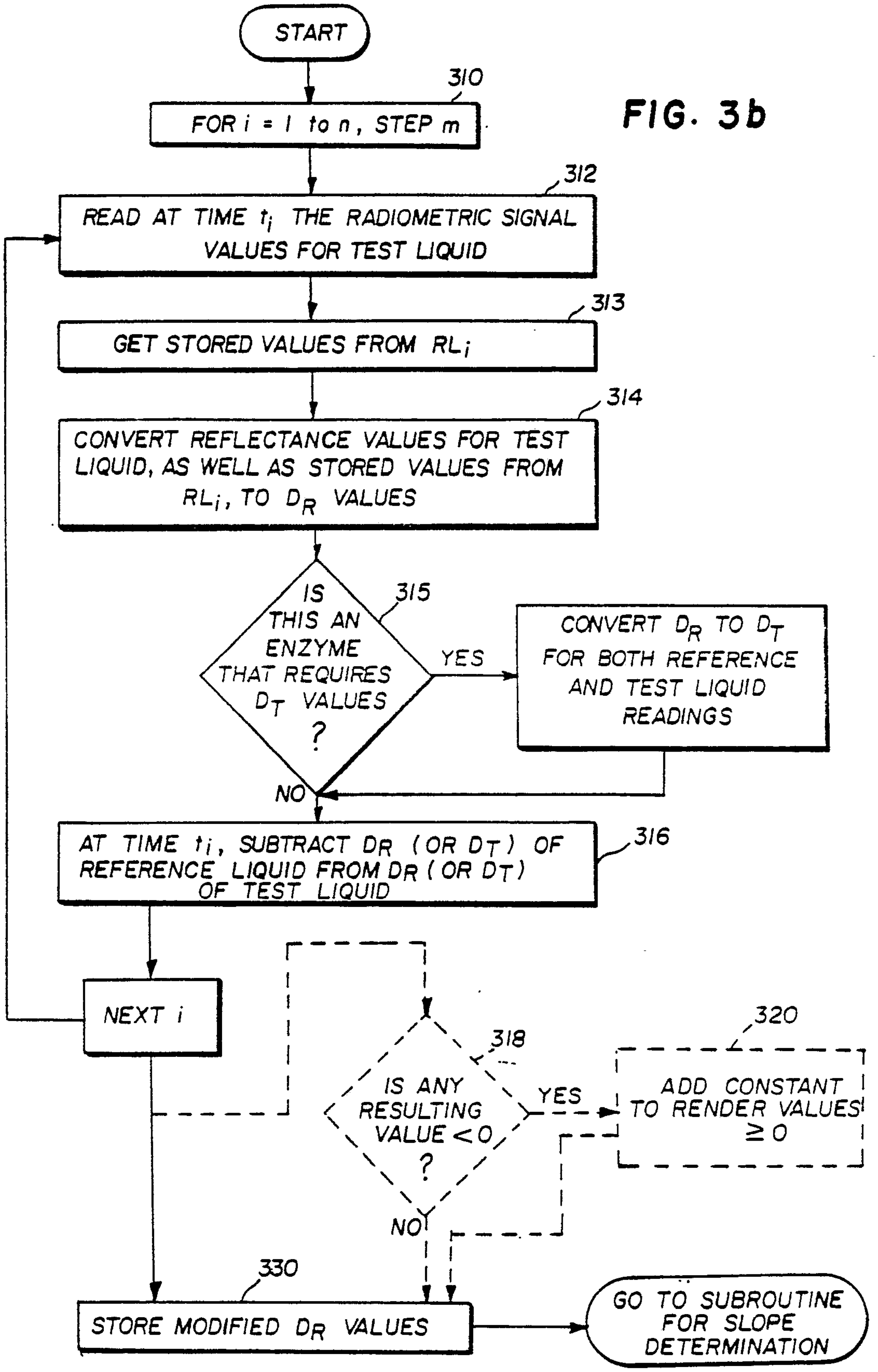
START
310
FOR i = 1 to n, STEP m
312
READ AT TIME $t_i$ THE RADIOMETRIC SIGNAL VALUES FOR TEST LIQUID
313
GET STORED VALUES FROM $RL_i$
314
CONVERT REFLECTANCE VALUES FOR TEST LIQUID, AS WELL AS STORED VALUES FROM $RL_i$, TO $D_R$ VALUES
315
IS THIS AN ENZYME THAT REQUIRES $D_T$ VALUES ?
YES
CONVERT $D_R$ TO $D_T$ FOR BOTH REFERENCE AND TEST LIQUID READINGS
NO
316
AT TIME $t_i$, SUBTRACT $D_R$ (OR $D_T$) OF REFERENCE LIQUID FROM $D_R$ (OR $D_T$) OF TEST LIQUID
NEXT i
318
IS ANY RESULTING VALUE < 0 ?
YES
320
ADD CONSTANT TO RENDER VALUES ≥ 0
NO
330
STORE MODIFIED $D_R$ VALUES
GO TO SUBROUTINE FOR SLOPE DETERMINATION

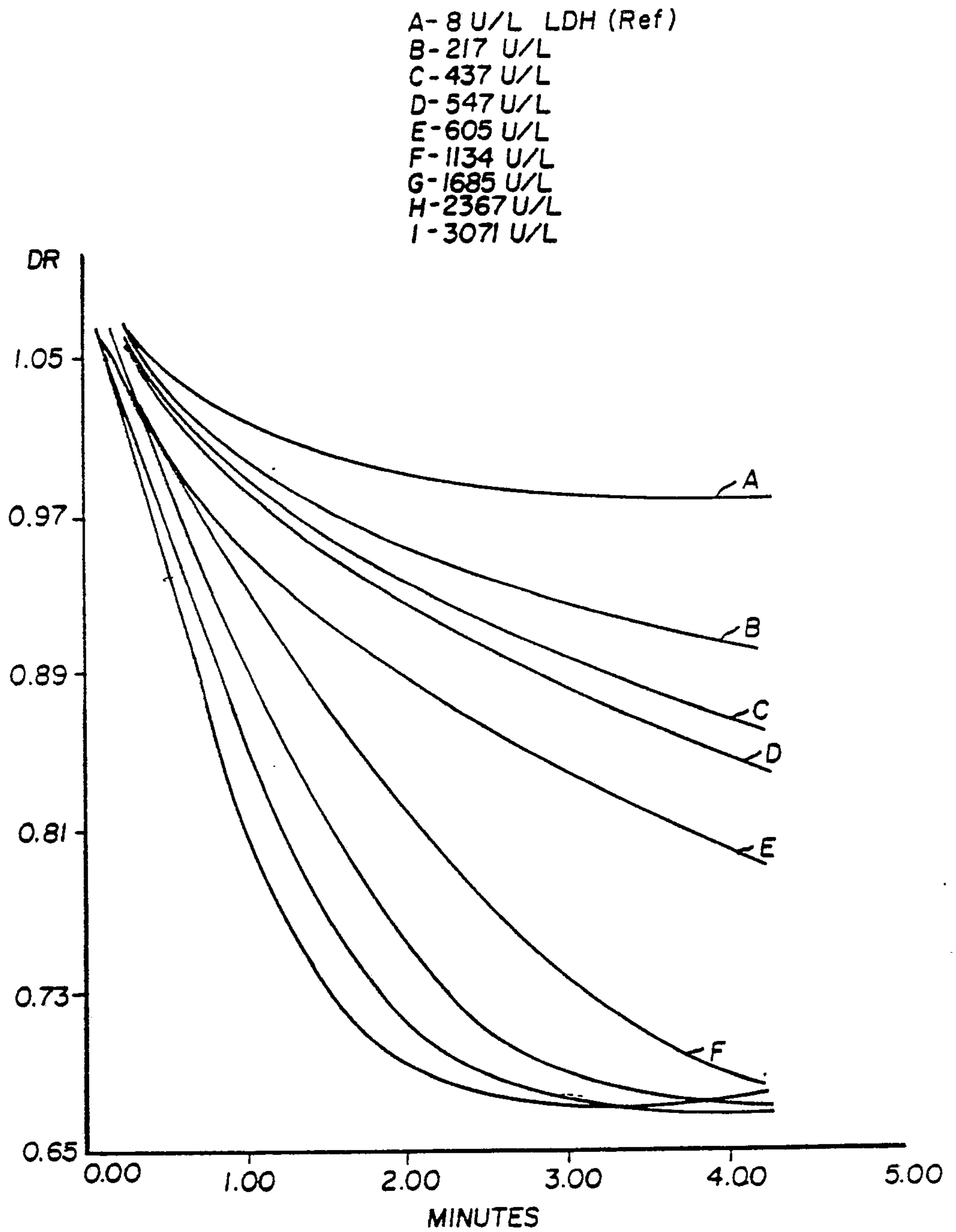
A- 8 U/L LDH (Ref)
B- 217 U/L
C- 437 U/L
D- 547 U/L
E- 605 U/L
F- 1134 U/L
G- 1685 U/L
H- 2367 U/L
I- 3071 U/L
DR
1.05
0.97
0.89
0.81
0.73
0.65
0.00
1.00
2.00
3.00
4.00
5.00
MINUTES
A
B
C
D
E
F

FIG. 4

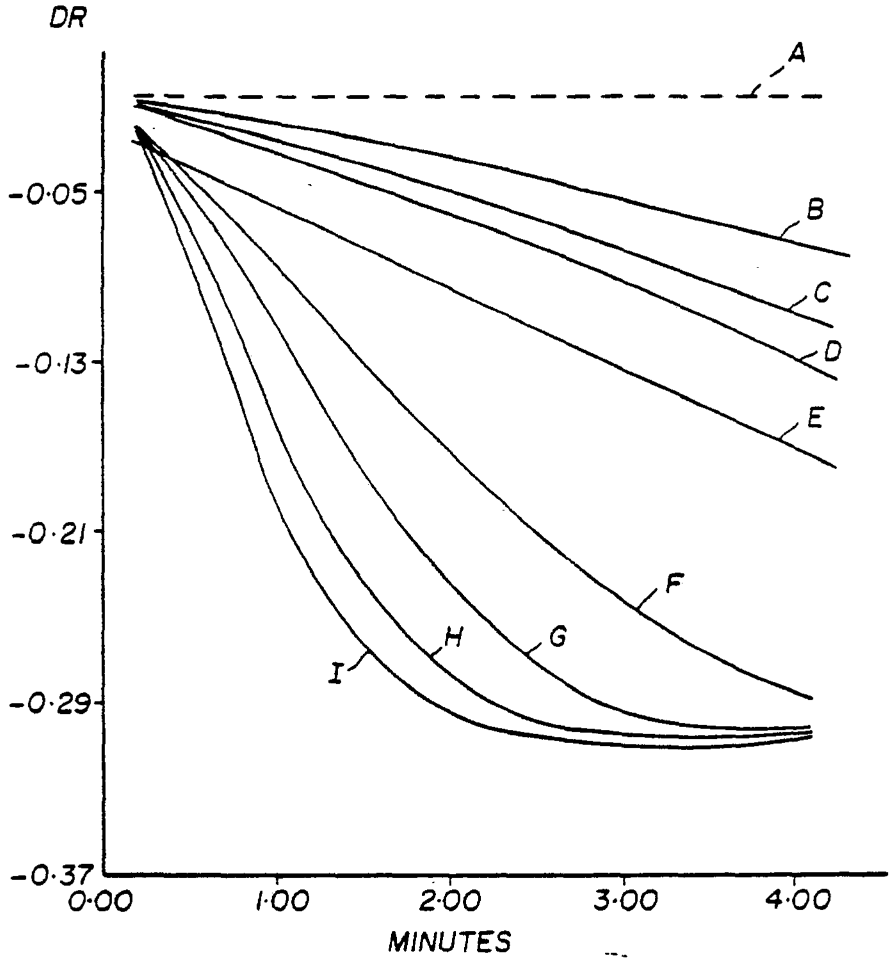


FIG. 5

A– 8 U/L LDH
B– 217 U/L
C– 437 U/L
D– 547 U/L
E– 605 U/L
F– 1134 U/L
G– 1685 U/L
H– 2376 U/L
I– 3071 U/L

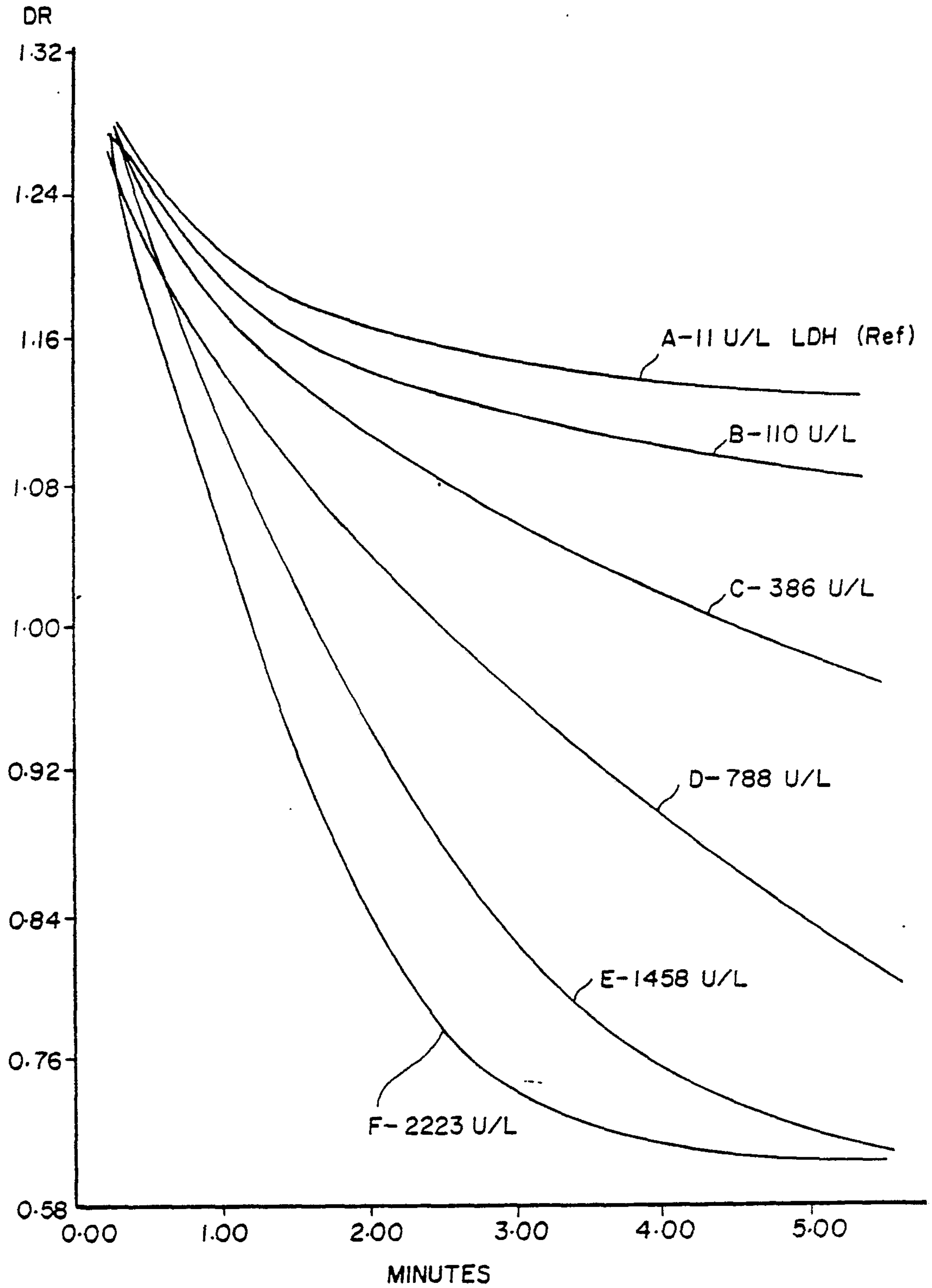
DR
1·32
1·24
1·16
1·08
1·00
0·92
0·84
0·76
0·58
0·00
1.00
2·00
3·00
4·00
5·00
MINUTES
A-11 U/L LDH (Ref)
B-110 U/L
C- 386 U/L
D-788 U/L
E-1458 U/L
F-2223 U/L

FIG. 6

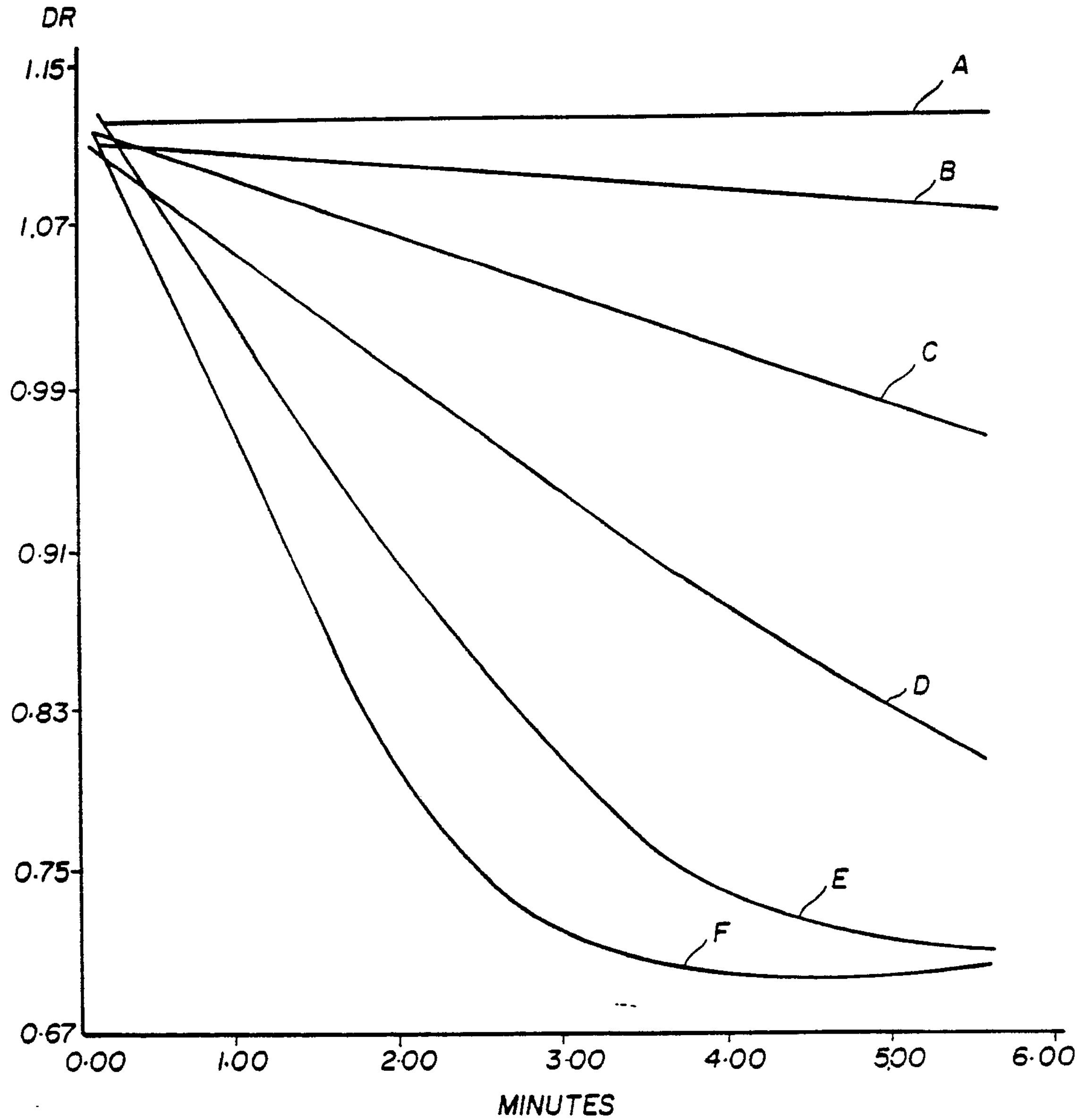


A– 11 U/L LDH
B– 110 U/L
C– 386 U/L
D– 788 U/L
E– 1458 U/L
F– 2223 U/L

FIG. 7

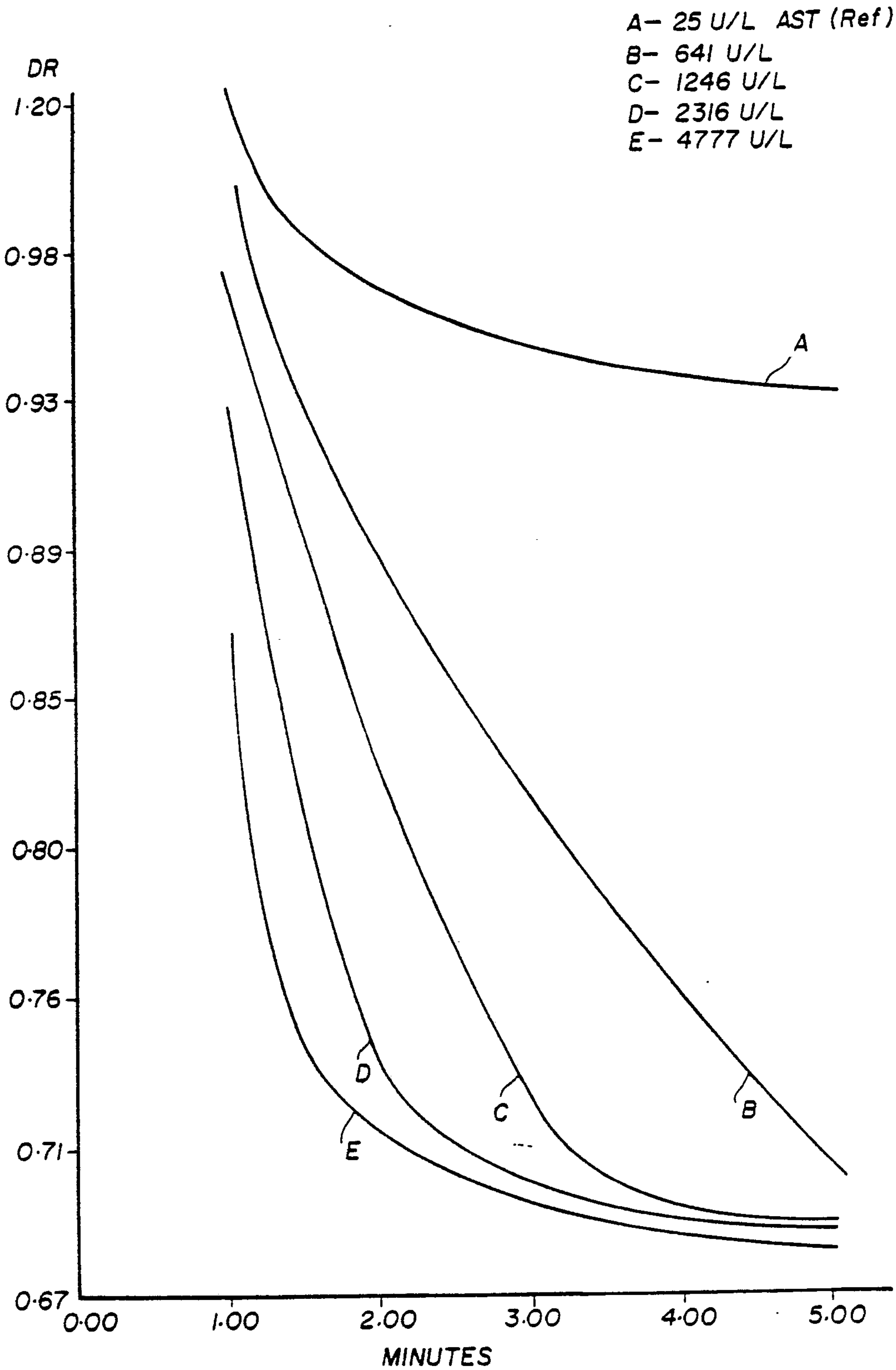
A– 25 U/L AST (Ref)
B– 641 U/L
C– 1246 U/L
D– 2316 U/L
E– 4777 U/L
DR
1·20
0·98
0·93
0·89
0·85
0·80
0·76
0·71
0·67
0·00
1·00
2·00
3·00
4·00
5·00
MINUTES
A
B
C
D
E

FIG. 8

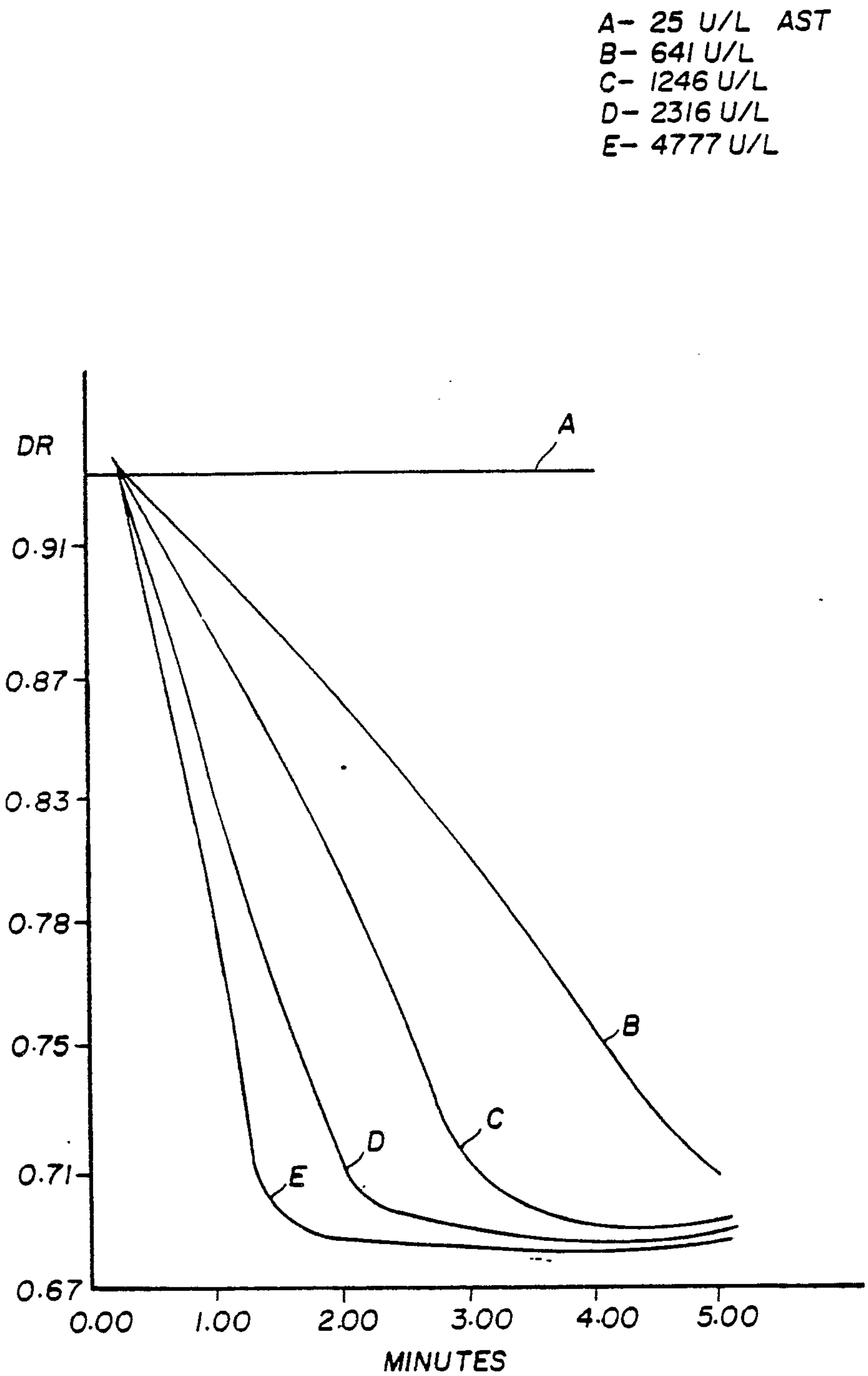
A– 25 U/L AST
B– 641 U/L
C– 1246 U/L
D– 2316 U/L
E– 4777 U/L
DR
A
0·91
0·87
0·83
0·78
0·75
0·71
0·67
B
C
D
E
0.00
1.00
2.00
3·00
4·00
5·00
MINUTES

FIG. 9

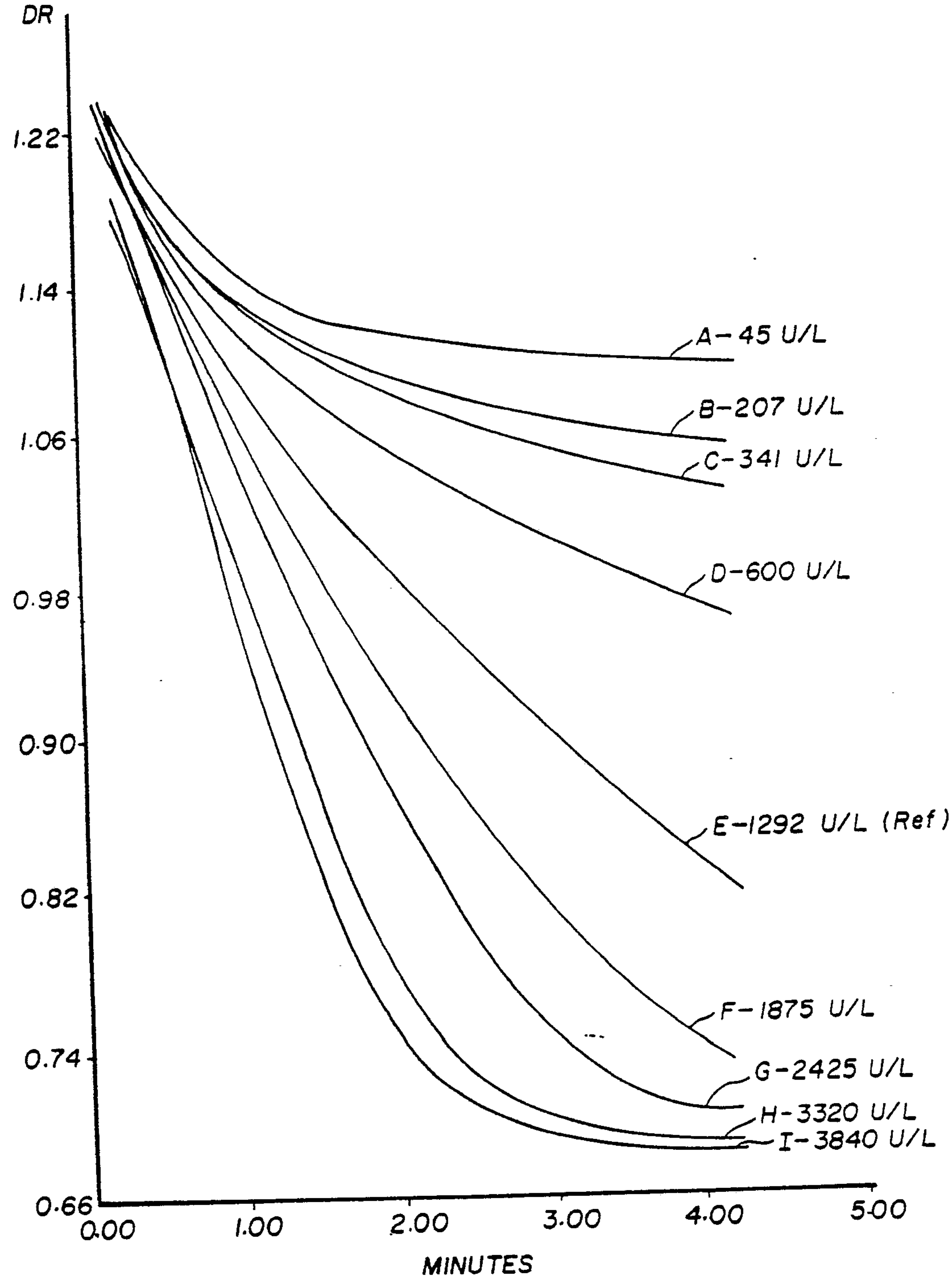
DR
1.22
1.14
1.06
0.98
0.90
0.82
0.74
0.66
0.00
1.00
2.00
3.00
4.00
5.00
MINUTES
A-45 U/L
B-207 U/L
C-341 U/L
D-600 U/L
E-1292 U/L (Ref)
F-1875 U/L
G-2425 U/L
H-3320 U/L
I-3840 U/L

FIG. 10

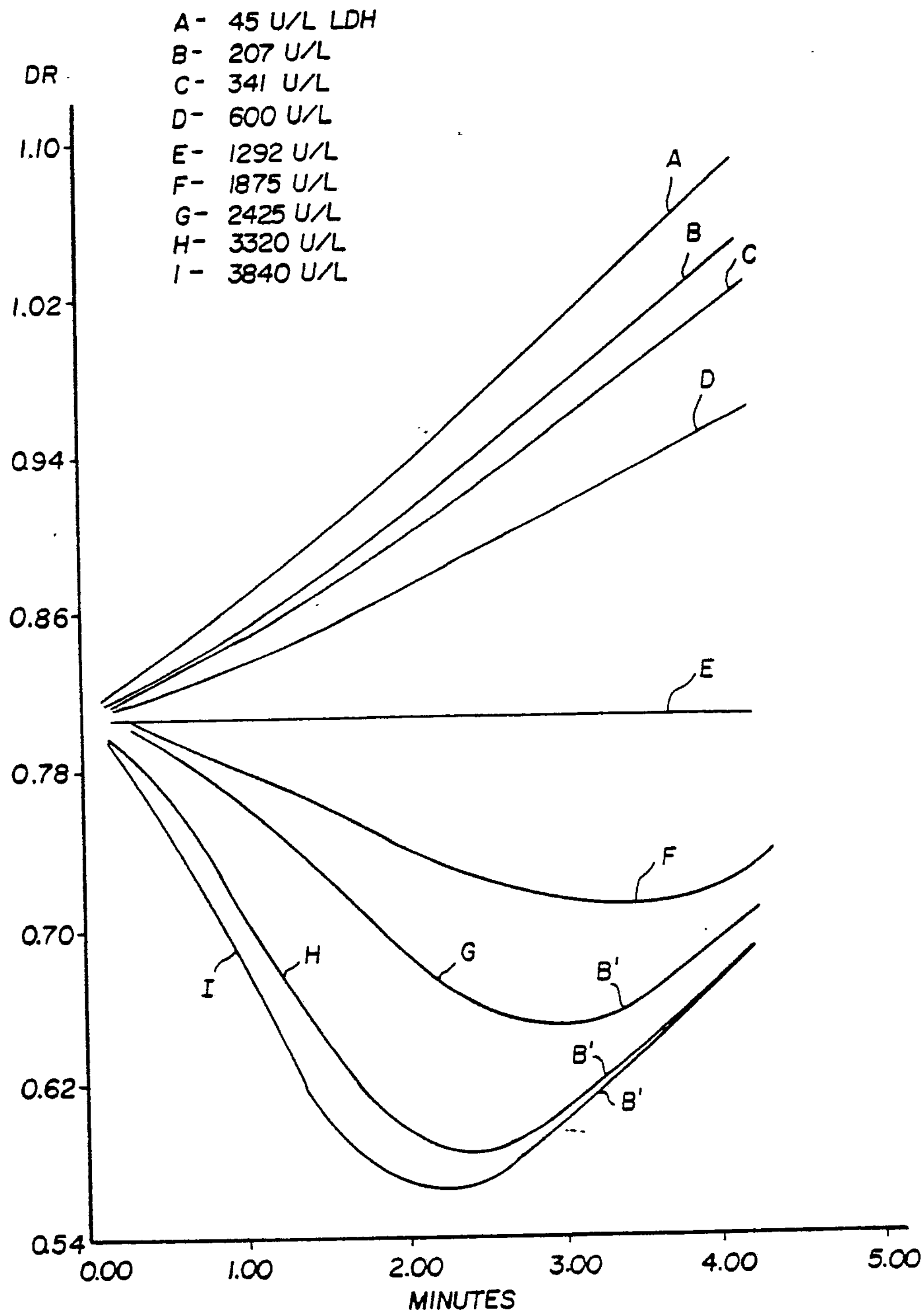
A- 45 U/L LDH
B- 207 U/L
C- 341 U/L
D- 600 U/L
E- 1292 U/L
F- 1875 U/L
G- 2425 U/L
H- 3320 U/L
I - 3840 U/L
DR
1.10
1.02
0.94
0.86
0.78
0.70
0.62
0.54
A
B
C
D
E
F
G
H
I
B'
B'
B'
0.00
1.00
2.00
3.00
4.00
5.00
MINUTES

FIG. 11

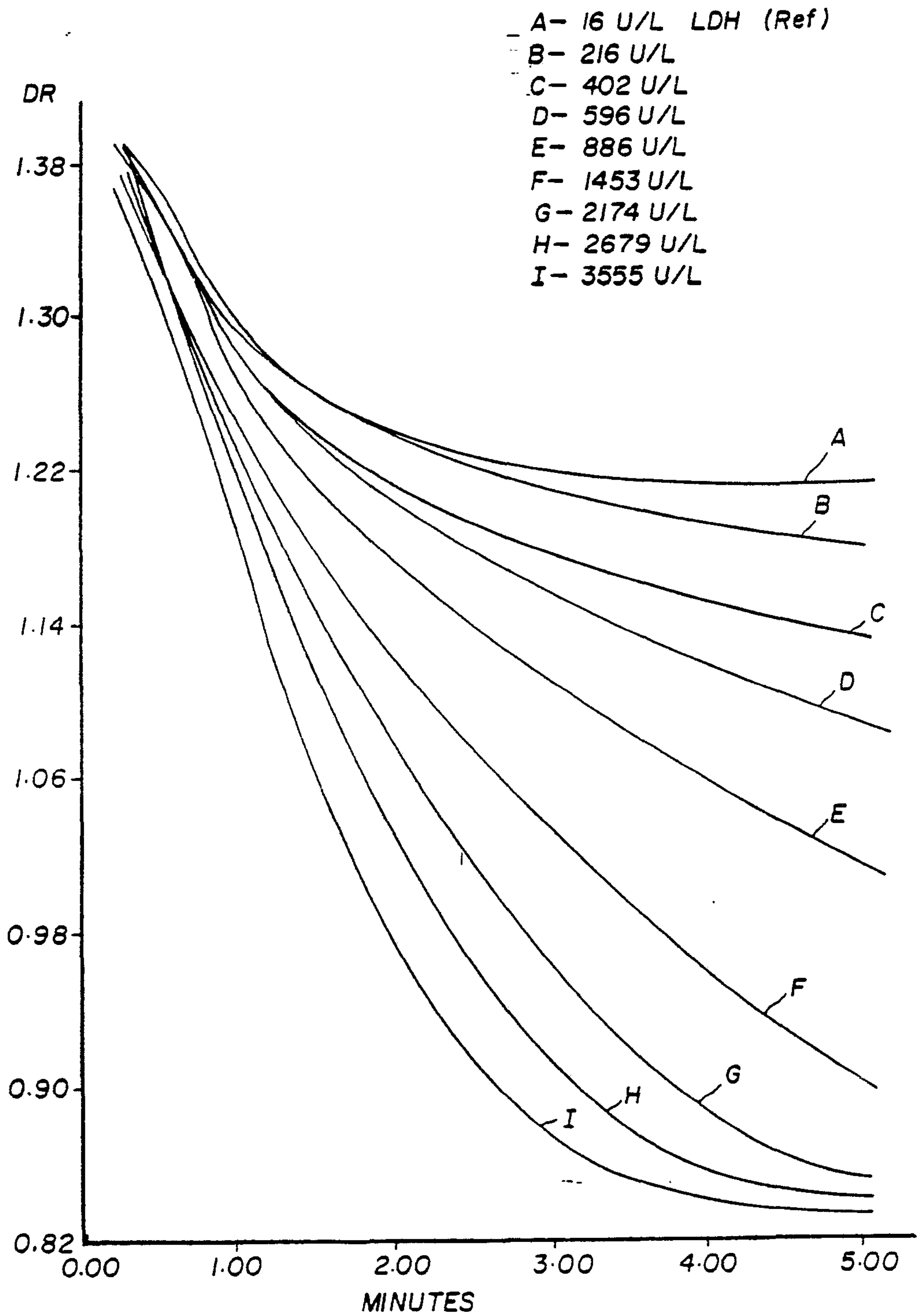
A– 16 U/L LDH (Ref)
B– 216 U/L
C– 402 U/L
D– 596 U/L
E– 886 U/L
F– 1453 U/L
G– 2174 U/L
H– 2679 U/L
I– 3555 U/L
DR
1.38
1.30
1.22
1.14
1.06
0.98
0.90
0.82
0.00
1.00
2.00
3.00
4.00
5.00
MINUTES
A
B
C
D
E
F
G
H
I

FIG. 12

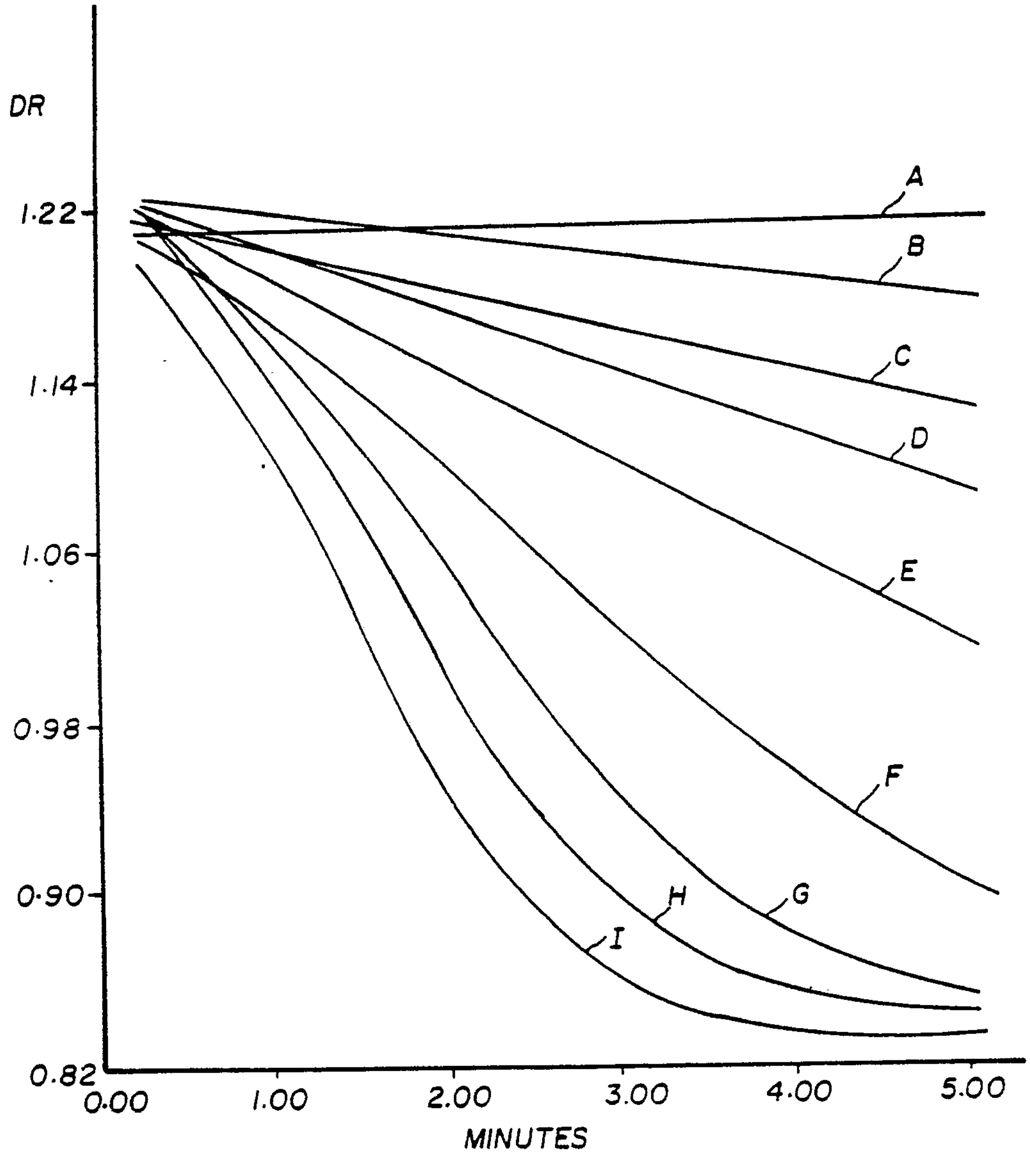


A– 16 U/L LDH
B– 216 U/L
C– 402 U/L
D– 596 U/L
E– 886 U/L
F– 1453 U/L
G.– 2174 U/L
H– 2697 U/L
I– 3555 U/L

FIG. 13

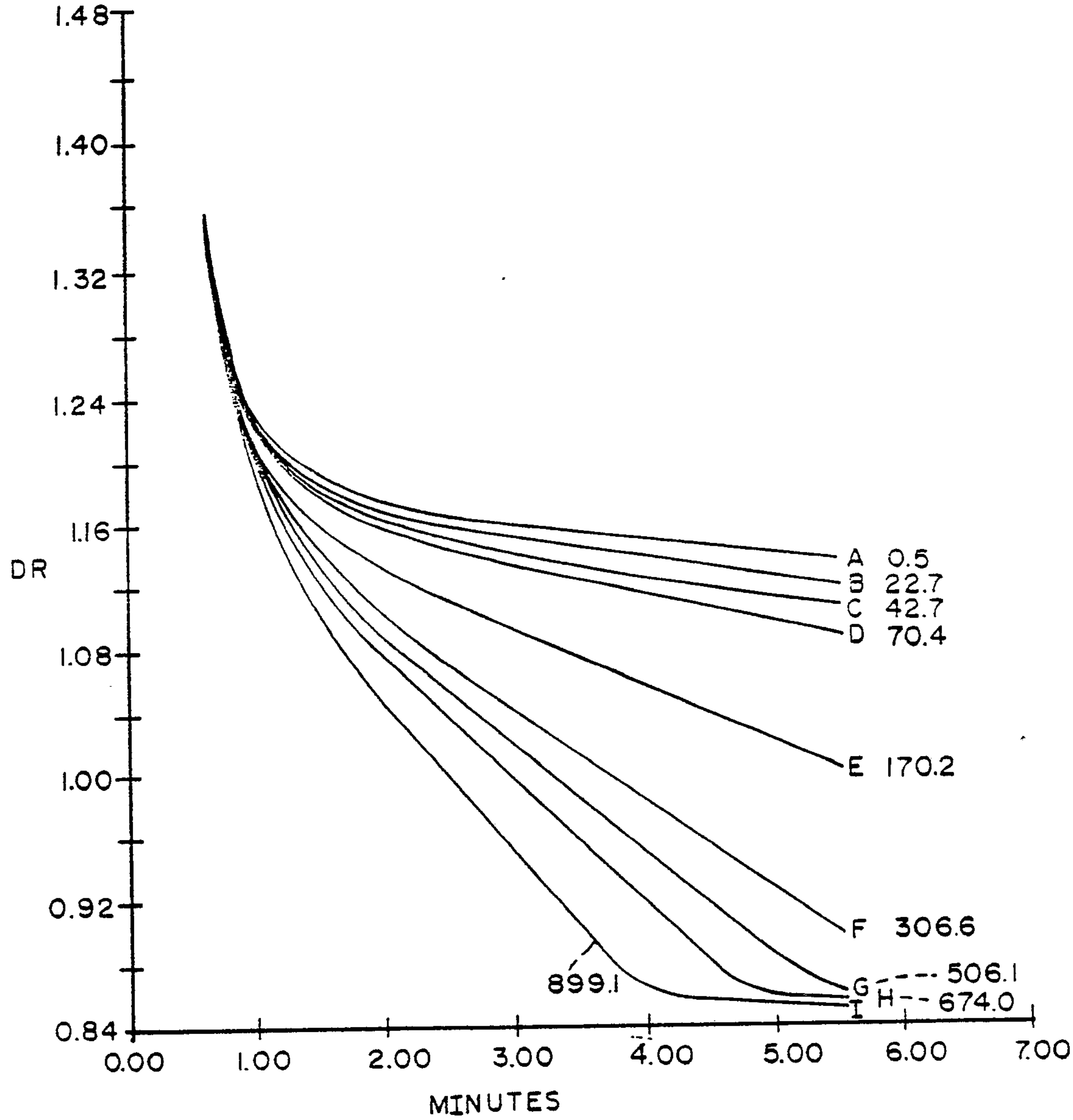
1.48
1.40
1.32
1.24
1.16
DR
1.08
1.00
0.92
0.84
0.00
1.00
2.00
3.00
4.00
5.00
6.00
7.00
MINUTES
A 0.5
B 22.7
C 42.7
D 70.4
E 170.2
F 306.6
G 506.1
H 674.0
I
899.1

FIG. 14

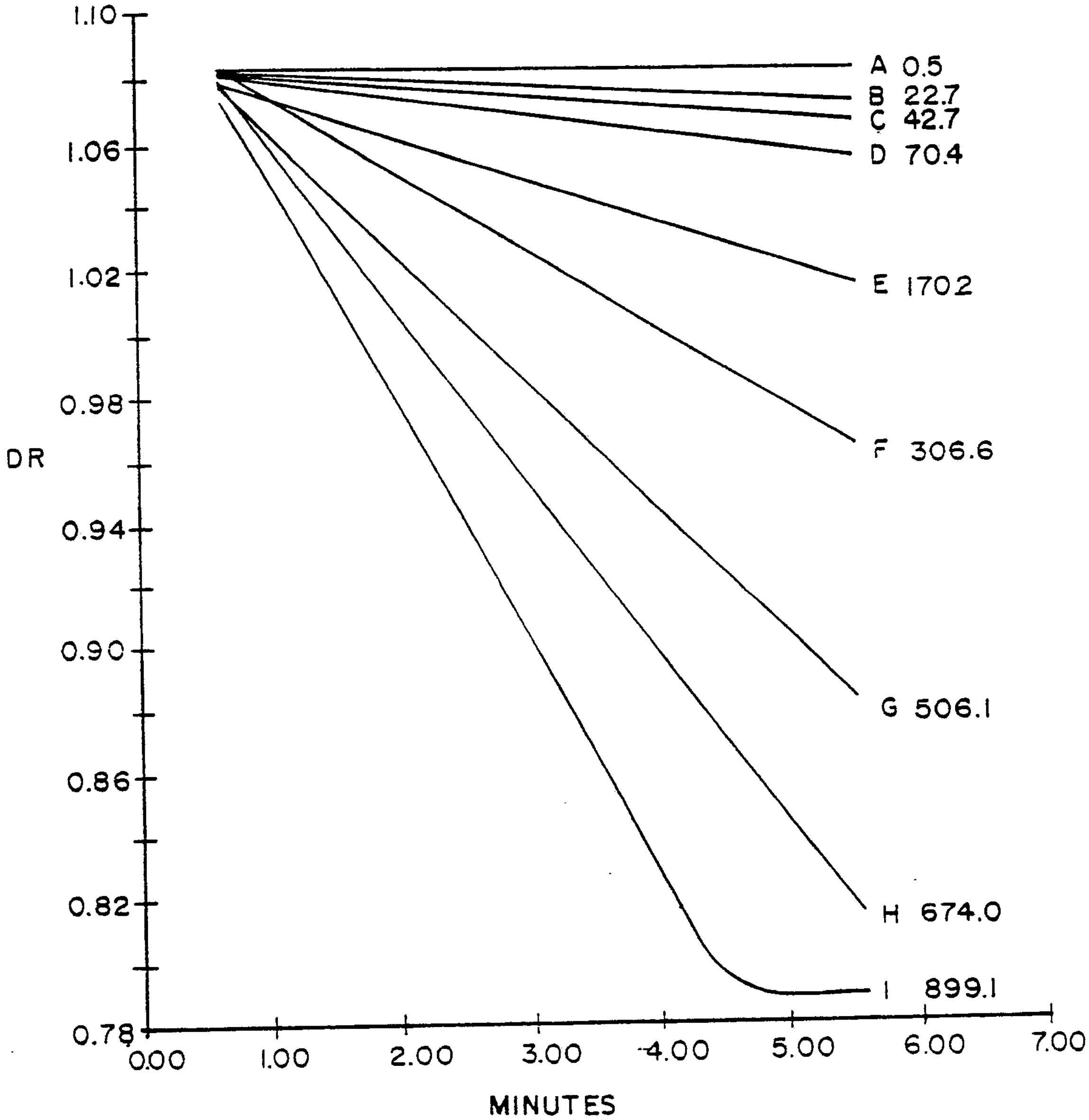
1.10
1.06
1.02
0.98
DR
0.94
0.90
0.86
0.82
0.78
0.00
1.00
2.00
3.00
4.00
5.00
6.00
7.00
MINUTES
A 0.5
B 22.7
C 42.7
D 70.4
E 170.2
F 306.6
G 506.1
H 674.0
I 899.1

FIG. 15